(19) Europäisches Patentamt
European Patent Office
Office européen des brevets

(11) Publication number: **0 427 690 A1**

# (12) EUROPEAN PATENT APPLICATION

(21) Application number: **90850346.9**

(22) Date of filing: **22.10.90**

(51) Int. Cl.5: **C07F 15/02**, C07D 487/22, A61K 31/555

(30) Priority: **07.11.89 SE 8903721**

(43) Date of publication of application: **15.05.91 Bulletin 91/20**

(84) Designated Contracting States: **AT BE CH DE DK ES FR GB GR IT LI LU NL SE**

(71) Applicant: **PROVIVO OY**
**Tekniikantie 17**
**SF-02150 Espoo(FI)**

(72) Inventor: **Lundell, Ralf Odert**
**Martendalsvägen 40 F 38**
**SF-01620 Vanda(FI)**
Inventor: **Mansson, Kristina Maria Vilhelmina**
**Stallmästarevägen 15 C**
**SF-02940 Espoo(FI)**
Inventor: **Tiainen, Eija Tuulikki**
**Kantelevägen 15 F 56**
**SF-00420 Helsingfors(FI)**

(74) Representative: **Inger, Lars Ulf Bosson**
**L + U INGER Patentbyra AB Garvaregatan 12**
**S-262 63 Ängelholm(SE)**

## (54) Process for the preparation of heme/hemin.

(57) The present invention relates to a process for the preparation of heme and/or hemin by extraction using a solvent of heme/hemin containing material, such as blood, haemoglobin, or heme/hemin sources, where in one utilizes, as a solvent, a solvent which has a considerably greater solubility to heme/hemin at an elevated temperature than at a lower temperature, and which solvent is liquid at the extraction and crystallization temperatures, and that the extraction is carried out at an acid or neutral pH, whereby preferably lower alkyl esters of formic acid, acetic acid, acetoacetic acid, 2-hydroxypropionic acid, 2-oxopropionic acid, malonic acid, maleic acid, malic acid and fumaric acid are used, the alkyl groups of the ester parts having 1 to 5 carbon atoms.



Xerox Copy Centre

# PROCESS FOR THE PREPARATION OF HEME/HEMIN

## Technical field

The present invention relates to a process for the preparation of heme and/or hemin by extraction from blood, haemoglobin, or other substances and products rich in heme.

The object of the present invention is to obtain a simple and rational process fcr the preparation of heme and/or hemin from globin and other heme and hemin rich substances and products to obtain heme or hemin with a good economical yield.

## Background of the invention

It is previously known that heme and hemin are substances that can be administered to humans and animals to prevent or heal iron deficiency anaemia.

Heme is thereby iron protoporphyrin which is present as prostetic group in haemoglobin, in cytocromes and in certain enzymes. The iron atom of heme can be present in a reduced ferro (+2) or in oxidized ferri (+3) form. In haemoglobin heme is bound via a histidine molecule whereby the release can take place via hemolysis and decomposition of this histidine binding. Hematin is also used as a name when heme is present in an alkaline environment and having $OH^-$ as counter ion.

Hemin is the name of the heme product that has $Cl^-$ as counter ion. In hemin the iron of the protoporphyrin has the $3^+$ value.

Other counter ions to the iron of protoporphyrin is i.a. $Br^-$ and $I^-$.

Hemin has the following structural formulae I

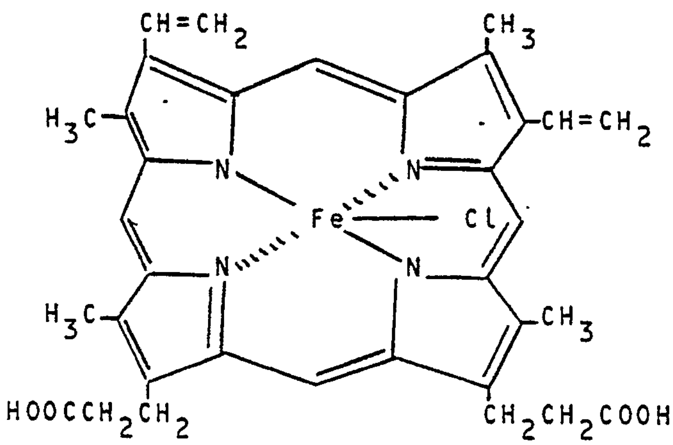

Hemin has the summary formulae $C_{34}H_{32}\ O_4N_4\ FeCl$.

In the haemoglobin molecule four heme groups are present. It is the heme groups that provide for the red colour of the blood and which are the active parts of haemoglobin for transporting oxygen.

It is already previously known to use hemin as such or as a derivative for the treatment of anaemia, therapeutically or prophylactically.

Preparation of heme/hemin can take place either via enzymatic cleavage of haemoglobin and isolation of the heme molecules (EP-A1-0 136 327) or via an acid hemolysis and extraction of heme. Heme has, however, as such a capability of aggregate and/or polymerize whereby problems arise at the isolation and optional crystallization of hemin.

The production of heme by means of extraction using acetone (the Labbe-Nishida method) or methyl ethyl ketone is previously known and the ketones are good solvents for heme/hemin. The drawback is that the ketones are not absolutely atoxic and that solubility reducing solvents (acetic acid) is needed for the crystallization of hemin crystals. The use of a 2 components system for the crystallization makes the production at an industrial scale more complicated.

JP-A2-283975/87 (Tosoh Corp.) relates to a process for the preparation of hemin where one utilizes

extraction with acetone of a complex of heme and hemin using polymers in an aqueous solution containing carboxy or sulphonyl groups. The crystallization takes place by the addition of acetic acid as well, before the solvent is being distilled off.

Besides the ketones above, lower alkanols have been used for the extraction of hemin from whole blood, haemoglobin or other substances rich in heme. In SE-C-7407882-5, and WO 81/02834 a process is described for the preparation of a concentrate of heme by extraction using lower alkanols, preferably ethanol, optionally in combination with ketones or esters, at pH >8.0, preferably 9.0 to 10.5, or in the presence of different imidazole derivatives from a mixture of heme and blood substance which has been obtained by acid hemolysis. The heme concentrate obtained contains 15 to 55 % of heme.

US-A-4,446,066 relates to a process for solvent extraction by means of acid and methanol and water, and/or ethanol, as well, whereby the phases rich in heme, and globin, respectively, are separated from each other using hydro cyclones. The extraction is carried out at 20 to 55°C.

JP-A-175425/87 (Toya Soda Mfg. Co.) discloses a process for the preparation of hemin via hematin (hemin hydroxide). Hereby, a heme complex is formed with carboxy and sulphonyl groups in an aqueous solution and the complexes are made alkaline before the extraction of the hematin to the organic solvent phase, such as methanol.

EP-A1-0 116 043 describes a process for separation of haemoglo bin into globin and heme fractions whereby heme is obtained as a complex with carboxy methyl cellulose (CMC).

None of the methods mentioned above can, in a simple and rational way, produce heme or hemin crystals, even if the yields using lower ketones at the extraction are good. Demands for a more simple and more rational industrial and economical method have thus been raised.

## Description of the present invention

It has now surprisingly been shown possible to be able to obtain economical and rational advantages by means of the present invention, which is characterized in that one utilizes, as solvent, a solvent which has a considerably greater solubility to heme/hemin at an elevated temperature than at a lower temperature, and which is liquid at the extraction and crystallization temperatures, and that the extraction is carried out at an acid or neutral pH.

Such solvents are lower alkyl esters having the following general formulae (I), (II), and (III)

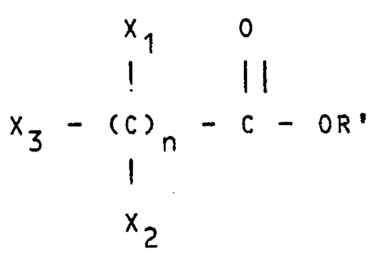 (I)

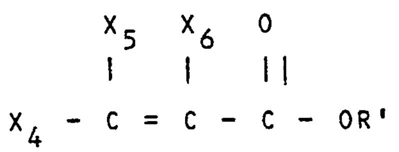 (II)

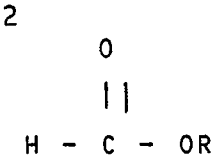 (III)

wherein $X_1$ is -H, -OH, a lower straight or branched alkyl group having 1 to 5 carbon atoms,

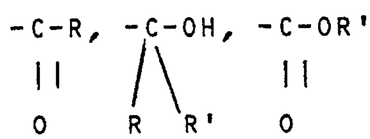

$X_2$ and $X_3$ are the same or different and are -H or a lower straight or branched alkyl group having 1 to 5 carbon atoms or $X_2$ and $X_3$ together form =O (oxo)

R is -H or a straight or branched alkyl group having 1 to 5 carbon atoms,

R′ is a lower straight or branched alkyl group having 1 to 5 carbon atoms,

n is an integer 1 or 2. When n = 2 the substituents $X_1$ and $X_2$ need not be the same on both carbon atoms. $X_4$ has the same meaning as $X_1$; $X_5$, and $X_6$ have the same meaning as $X_2$.

Such esters are the formic acid esters, acetic acid esters, lactic acid esters, acetoacetic acid esters, beta-hydroxy butyric acid esters, malonic acid esters, pyruvic acid esters, and maleic acid esters.

Preferred esters are the esters of 2-hydroxypropionic acid (lactic acid) or 2-oxopropionic acid (pyruvic acid), whereby the alkyl groups have 1 to 5 C.

Such esters are methyl formate, ethyl formate, n-propyl formate, isopropyl formate, n-butyl formate, tert.butyl formate, amyl formate, methyl acetate, ethyl acetate, n-propyl acetate, isopropyl acetate, tert.butyl acetate, and amyl acetate, 2-OH-propionic acid methyl ester, 2-OH-propionic acid ethyl ester, 2-OH-propionic acid isopropyl ester, 2-OH-propionic acid n-propyl ester, 2-OH-propionic acid tert.butyl ester, 2-OH-propionic acid amyl ester, 2-oxo-propionic acid methyl ester, 2-oxo-propionic acid ethyl ester, 2-oxo-propionic acid isopropyl ester, methyl acetoacetate, ethyl acetoacetate, isopropyl acetoacetate, amyl acetoacetate, 3-OH-butyric acid methyl ester, 3-OH-butyric acid ethyl ester, 3-OH-butyric acid isopropyl ester, methyl malonate, ethyl malonate, isopropyl malonate, amyl malonate, methyl maleate, ethyl maleate, isopropyl maleate, and amyl maleate.

Heme containing raw materials are whole blood, the haemoglobin fraction from blood (human or animalic), heme-CMC-complex, con centrate of heme according to WO 81102834, enzyme degraded fractions of haemoglobin, and other heme/hemin containing products.

In order to obtain an acid pH in the extracted raw material it might be necessary to add an acid to the starting material, such as hydrochloric acid, sulphuric acid, nitric acid or an organic acid, such as acetic acid, propionic acid, citric acid or other relatively inexpensive acid.

In general the present process means an extraction at an elevated temperature, suitably above 400° C, preferably above 100° C and suitably in an acidic environment, whereby heme is transferred into the organic phase very rapidly, addition of chloride ions to the formation of hemin, and cooling to ambient temperature or lower, or distillation off of part of the the amount of solvent, whereby hemin crystallizes. If the hemin should hesitate in crystallizing seed crystals can be added, but normally this is not necessary.

As the extraction takes place very rapidly, within the course of some minutes, the extraction can be carried out as a simple batchwise, or as a continuous counter-current process (or optionally co-current process) using a short contact zone. Complicated equipment is thus not needed. The yield of heme is normally, directly after a one step extraction, as crystal pure product 40 to 80 %. The yields can be improved with some percent units partly by further extractions, partly by further distillation off/concentration and cooling of the mother liquor.

Prior to the crystallization the extraction solution is filtered free from any solid substance. such as globin and/or other denaturated proteins.

In stead of filtration centrifugation can be used. Filtration or centrifugation are suitably carried out at extraction tem perature in order to avoid precipitation of hemin crystals in the equipment.

For the cleavage of the heme-globin bound a hemolysis should be utilized, whereby pH is lowered to <3 by addition of suitably HCl. If one starts with heme-CMC the complex is most often broken directly at the dissolution in the said solvent.

The relationship between solvent and heme/hemin in the raw material shall be >100:1, suitably >1000:5, preferably >1000:2. The yield of hemin increases with increasing amount of solvent.

To obtain crystalline hemin $Cl^-$ ions are added, suitably in the form of readily dissolvable chloride salts or HCl. Other counter ions are $Br^-$ and $I^-$ and the corresponding heme compounds are thus iron protoporphyrin bromide and iodide.

The invention will be described more in detailö in the examples given however, without being restricted thereto.

EXAMPLE 1.

10 g heme-CMC complex obtained from the process according to EP-A1-0 116 043 (about 2 % of heme) were provided with and extracted with 200 ml of methyl lactate at a temperature of 116°C for 10 min. The extraction solution was filtered in order to remove filtering aids, residual proteins and CMC present in the raw material. The yield in the extraction step was 66 %. To 100 ml of the above extraction solution 51 mg of $FeCl_3 x 6H_2O$ were added, and 67 ml of the solvent were evaporated, whereby the hemin crystals precipitated. The yield of the crystalliza-tion was 86 %.

It was verified using the pyridine hemocrome method (Kevin M. Smith, Porphyrins and Metalloporphyrins, Elsevier Scientific Publ. Co. (1975) p. 804, (J.H. Fuhrhop & K. M. Smith)) that the crystals consisted

of heme.

EXAMPLE 2.

10 g of heme-CMC complex (1.9 % heme) were extracted with 200 ml of isopropyl lactate at a temperature of 114°C for 10 min. The extraction solution was filtered as in Ex. 1 above. Extraction yield: 71 %. To 100 ml of the above extraction solution 47 mg of $FeCl_3x6H_2O$ were added and the solution was cooled to room temperature (22°C), whereby 57.5 mg of hemin crystals were obtained.

EXAMPLE 3.

3 g of heme-CMC complex were provided with and extracted with 42 ml of ethyl lactate at a temperature of 110°C for 5 min. The extraction solution was filtered as in Ex. 1 above. Extraction yield: 88 %. To the extraction solution 46 mg of $FeCl_3x6H_2O$ were added and the solution was cooled to +5°C, whereby hemin crystallized. Yield of the crystallization: 66 %.

EXAMPLE 4.

20 g of heme-CMC complex acc. to Ex. 1 were extracted with 400 ml of ethyl acetoacetate at 104°C for 5 min. Extract ion yield: 39 %. After filtration in accordance with Ex. 1 the filtrate was concentrated to 20 ml and 100 mg of $FeCl_3x6H_2O$ were added. Yield of the crystallization: 85 %.

EXAMPLE 5.

10g of heme-CMC complex were provided with 300 ml ethyl maleate at 100°C so that the water present (7 g) was distilled off, whereupon the temperature was raised to 130°C. The extract ion solution was filtered in accordance with Ex. 1 above. To the filtrate 10,3 mg of $FeCl_3x6H_2O$ were added. The extraction yield was 39 %. At cooling to room temperature (22°C) 42.7 mg of hemin crystals were obtained, which gave a crystallization yield of 55 %.

EXAMPLE 6.

10g of heme-CMC complex were provided with 400 ml ethyl malonate at 100°C, whereupon the temperature was raised to 137°C.

The extraction solution was filtered in accordance with Ex. 1 above, and 10 mg of $FeCl_3x6H_2O$ were added. The extraction yield was 27 %. At cooling to room temperature hemin crystals were obtained and the crystallization yield was 38 %.

EXAMPLE 7.

5 g of heme-CMC complex were provided with 100 ml ethyl pyruvate at 900°C, and the heme was extracted at this temperature for 10 min. Extraction yield was 47 %. After filtration in accordance with example 1 above 5,3 mg of $FeCl_3x6H_2O$ were added and after cooling hemin crystals were separated from the mother liquor by centrifugation. The crystallization yield was 71 %.

EXAMPLE 8.

3 g of heme-CMC-complex were extracted with 90 ml of ethyl-3OH-butyrate at 130°C for 5 min. The extraction solution was filtered acc. to Ex. 1 above. Extraction yield 79 %. 16 mg of $FeCl_3x6H_2O$ were added to the extraction solution whereupon the solution was cooled to room temperature (22°C), whereby hemin crystals precipitated. Crystallization yield: 53 %.

EXAMPLE 9.

59 g of red blood cells were diluted with 250 ml of weak HCl solution (pH ( 2) and the pH of the final solution was adjusted to 1.5, Hemolysis was carried out at 40°C for 30 min. The pH of the solution was increased to 3 and the heme precipitate obtained was centrifuged at 11000 rpm. 15 g of this centrifuged precipitate were extracted with 150 ml isopropyl lactate at 110 °C for 5 min. The extract ion solution was filtered in acc. with Ex. 1 above. Extraction yield: 77 %. 88 ml of the solvent were evaporated whereby the hemin crystals precipitated. Crystallization yield: 70 %.

EXAMPLE 10.

To 10 g of haemoglobin 50 ml of $H_2O$ were added and the pH of the solution was lowered to <3 using HCl. To this solution 100 ml of ethyl acetoacetic acid were added having the temperature 120 °C. The extraction was carried out at 110°C for 10 min. The water was evaporated under vacuo, whereupon the extraction solution was filtered, and 107 mg of $FeCl_3x6H_2O$ were added. The extraction solution was cooled to +5°C, whereby hemin crystals precipitated.

## Claims

1. Process for the preparation of heme and/or hemin by solvent extraction from blood, haemoglobin, or other substances and products rich in heme, characterized in that one utilizes, as a solvent, a solvent selected from the group of solvents having the general formulae (I), (II), and (III)

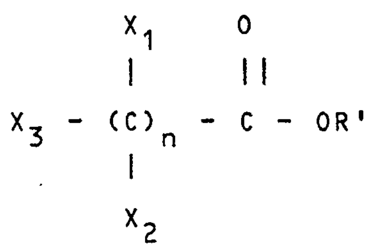
(I)

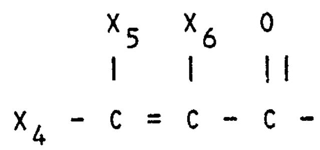
(II)

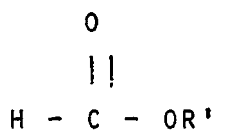
(III)

wherein $X_1$ is -H, -OH, a lower straight or branched alkyl group having 1 to 5 carbon atoms,

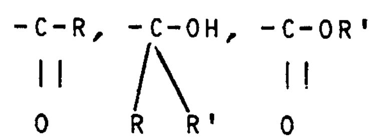

$X_2$ and $X_3$ are the same or different and are -H or a lower straight or branched alkyl group having 1 to 5 carbon atoms or X2 and $X_3$ together form =O (oxo)
R is -H or a straight or branched alkyl group having 1 to 5 carbon atoms,
R' is a lower straight or branched alkyl group having 1 to 5 carbon atoms,
n is an integer 1 or 2. When n=2 the substituents $X_1$ and $X_2$ need not be the same on both carbon atoms.
$X_4$ has the same meaning as $X_1$; $X_5$, and $X_6$ have the same meaning as $X_2$, which solvent is liquid at the extraction and crystallization temperatures, and that the extraction is carried out at an acid or neutral pH.
2. Process according to claims 1, characterized in that the solvent is a lower alkyl ester of formic acid, acetic acid, 2-hydroxypropionic acid, 2-oxopropionic acid, acetoacetic acid, 3-OH-butyric acid, malonic acid, maleic acid, whereby the alkyl groups of the ester parts have 1 to 5 carbon atoms,

3. Process according to claim 1, characterized in that the extraction is carried out at an elevated temperature.

4. Process according to claim 3, characterized in that the extraction is carried out at at least 40°C, preferably above 100 °C.

5. Process according to claim 1, characterized in that the ratio solvent: heme/hemin is greater than 100:1, preferably greater than 1000:5, more preferably greater than 1000:2 during the extraction.

6. Process according to one or more of claims 1 to 5, characterized in that chloride ions are added in order to improve the crystallization of hemin.

7. Process according to claim 6, characterized in that the chloride ions are added in the form of HCl, ferri chloride, and/or ferro chloride.

European Patent Office

# EUROPEAN SEARCH REPORT

Application number
EP 90850346.9

## DOCUMENTS CONSIDERED TO BE RELEVANT

| Category | Citation of document with indication, where appropriate, of relevant passages | Relevant to claim | CLASSIFICATION OF THE APPLICATION (Int Cl 5) |
|---|---|---|---|
| A | ABDERHALDEN, BIOCHEMISCHES HAND-LEXIKON, vol. VI, 1911 * pages 234-239 * --- | 1-7 | C 07 F 15/02<br>C 07 D 487/22<br>A 61 K 31/555 |
| A | ABDERHALDEN, BIOCHEMISCHES HAND-LEXIKON, vol. IX, 1915 * pages 342-345 * --- | 1-7 | |
| A | EP-A-0 236 971 (BASF) 16 September 1987 * The whole document * --- | 1-7 | |
| | | | TECHNICAL FIELDS SEARCHED (Int Cl 5) |
| | | | C 07 F<br>C 07 D<br>A 61 K |

The present search report has been drawn up for all claims

| Place of search | Date of completion of the search | Examiner |
|---|---|---|
| STOCKHOLM | 09-01-1991 | KARLSSON G. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document